(19) Europäisches Patentamt
European Patent Office
Office européen des brevets

(11) Numéro de publication: **0 499 755 A1**

(12) **DEMANDE DE BREVET EUROPEEN**

(21) Numéro de dépôt: **91400415.5**

(22) Date de dépôt: **18.02.91**

(51) Int. Cl.5: **C07C 229/50**, A61K 31/24, A61K 31/95, C07C 271/24

(43) Date de publication de la demande: **26.08.92 Bulletin 92/35**

(84) Etats contractants désignés: **IT**

(71) Demandeur: **MIDY S.p.A.**
**Via Piranesi 38**
**I-20137 Milano(IT)**

(72) Inventeur: **Badone, Domenico**
**Via Andreoli No. 2**
**I-21056 Induno Olona (Varese)(IT)**
Inventeur: **Guzzi, Umberto**
**Via Don Gnocchi 28**
**I-20010 Milano(IT)**

(74) Mandataire: **Gillard, Marie-Louise et al**
**Cabinet Beau de Loménie 55, Rue d'Amsterdam**
**F-75008 Paris(FR)**

(54) **Nouvelles phényléthanolaminotétralines, procédé pour leur préparation, intermédiaires dans ce procédé et compositions pharmaceutiques les contenant.**

(57) Phenyléthanolaminotétralines de formule (I)

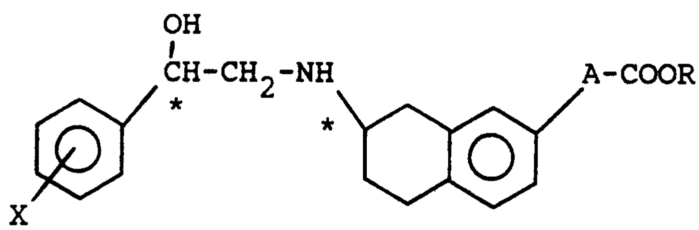

(I)

dans laquelle

X représente un atome d'hydrogène, un atome d'halogène, un groupe ($C_1$-$C_4$)alkyle ou un groupe trifluorométhyle,

A représente une liaison entre le groupe -COOR et le noyau tétralinique, un groupe ($C_1$-$C_4$)alkylène ou un groupe ($C_2$-$C_4$)alcénylène, et

R est un atome d'hydrogène ou un groupe ($C_1$-$C_4$)alkyle, et leurs sels, sont notamment préparés par réaction d'un époxyde de formule (IIa)

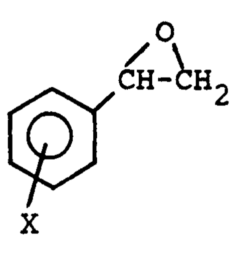

(IIa)

avec une tétraline de formule (III)

EP 0 499 755 A1

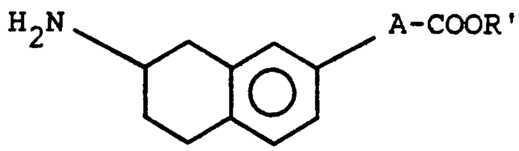

(III)

dans laquelle A est tel que defini ci-dessus et R' est un groupe ($C_1$-$C_4$)alkyle, éventuellement suivie par une hydrolyse basique pour obtenir les composés (I) où R est l'hydrogène.

Les nouveaux composés ont des propriétés très intéressantes en tant qu'agents antidépresseurs et spasmolytiques intestinaux.

Les nouveaux intermédiaires de formule (III) sont aussi revendiqués.

La présente invention concerne des nouveaux dérivés phényléthanolaminotétraliniques, le procédé pour leur préparation, les intermédiaires dans ce procédé ainsi que les compositions pharmaceutiques renfermant les nouveaux produits.

Des dérivés phényléthanolaminotétraliniques caractérisés par la présence d'un atome d'oxygène lié au noyau tétralinique ont été décrits, en tant qu'agents lipolytiques et modulateurs de la motricité intestinale, dans les demandes de brevet EP-A-211721 et EP-A-383686.

On a maintenant trouvé que les phényléthanolaminotétralines ayant un groupe carboxy ou carbalcoxy lié à la position 7-du noyau 1,2,3,4-tétrahydronaphtalènique soit directement soit via un groupe alkylène ou alcénylène inférieur, ont des propriétés pharmacologiques très intéressantes en tant qu'agents antidépresseurs. Ainsi la présente invention concerne en un de ses aspects les phényléthanolaminotétralines de formule (I) :

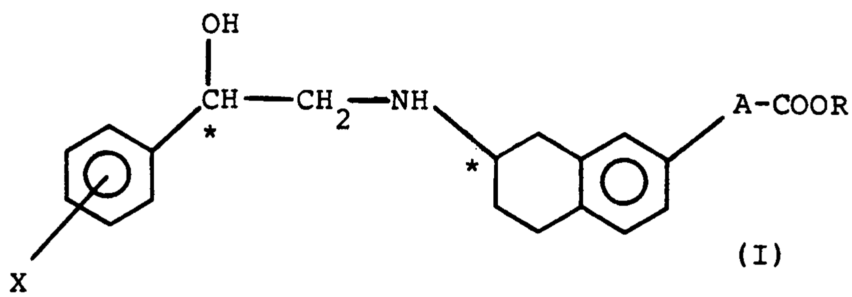

(I)

dans laquelle

X représente un atome d'hydrogène, un atome d'halogène, un groupe ($C_1$ - $C_4$)alkyle ou un groupe trifluorométhyle,

A représente une liaison entre le groupe -COOR et le noyau tétralinique, un groupe ($C_1$-$C_4$)alkylène, ou un groupe ($C_2$-$C_4$)alkénylène, et

R représente un atome d'hydrogène ou un groupe ($C_1$-$C_4$)alkyle, et leurs sels.

Dans la présente description, les termes "($C_1$-$C_4$)alkyle" et "($C_1$-$C_4$)alkylène" designent un radical monovalent et divalent, respectivement, d'un hydrocarbure saturé à chaîne droite ou ramifiée qui peut contenir de 1 à 4 atomes de carbone. De préférence le terme "($C_1$-$C_4$)alkyle" désigne un radical méthyle ou éthyle, tandis que le terme "($C_1$-$C_4$)alkylène" désigne de préférence un radical méthylene, éthylène ou propylène qui peut être éventuellement substitué par méthyle.

Le terme "($C_1$-$C_4$)alcénylène" identifie un radical divalent d'un hydrocarbure à chaîne droite ou ramifiée contenant de 2 à 4 atomes de carbone et une liaison double. De préférence le terme "($C_2$-$C_4$)alcénylène" représente un radical vinylène, ou 1,3-propylène.

Le terme "halogène" comprend les quatre halogènes : fluore, chlore, brome et iode, le chlore étant préféré. Les sels des composés de formule (I) selon la présente invention comprennent aussi bien les sels d'addition avec des acides minéraux ou organiques pharmaceutiquement acceptables tels que le chlorhydrate, le bromohydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I), tels que le picrate, l'oxalate ou les sels d'addition avec des acides optiquement actifs, par exemple les acides camphorsulfoniques et les acides mandéliques ou mandéliques substitués.

De plus, lorsque le composé de formule (I) possède un groupe carboxy libre (R = H) les sels comprennent aussi les sels avec des bases minérales, de préférence celles avec des métaux alcalins tels que le sodium ou le potassium, ou avec des bases organiques, comme le trométamol.

Dans la formule (I) ci-dessus, les deux atomes de carbone asymétriques sont marqués par une astérisque. Tous les composés de formule (I) peuvent donc exister sous forme d'au moins quatre isomères stériques differents, (R,R), (R,S), (S,S) et (S,R). Les stéréoisomères optiquement purs, ainsi que les mélanges des deux, trois ou tous les quatre isomères, dans une proportion quelconque, font partie de la présente invention. D'autres centres d'asymétrie pourraient être présents dans le radical A. De même, les stéréoisomères provenant de la présence d'un centre chiral additionel et leurs mélanges font partie de l'invention.

Des composés préférés dans la présente invention comprennent les composés de formule (I) dans laquelle X réprésente un atome d'hydrogène ou un atome d'halogène ou un groupe trifluorométhyle en position 3, et leurs sels.

D'autres composés préférés de la présente invention comprennent les composés de formule (I) dans laquelle A représente une liaison entre le groupe -COOR et le noyau tétralinique, un groupe alkylène à 1 ou 2 atomes de carbone ou un groupe alcénylène à 2 atomes de carbone, et leurs sels.

Des composés de la présente invention particulièrement avantageux comprennent les composés de formule (I) dans laquelle X est un atome d'halogène en position 3, A représente une liaison entre le groupe -COOR et le noyau tétralinique, ou un groupe alkylène à 1 ou 2 atomes de carbone, et R représente un atome d'hydrogène ou un groupe méthyle ou éthyle, et leurs sels.

Les composés de formule (I) peuvent être préparés par un procédé caractérisé en ce qu'on traite un composé de formule (II) :

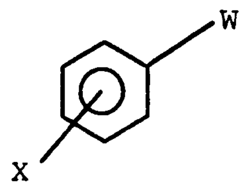

(II)

dans laquelle X est tel que défini ci-dessus et le radical -W réprésente un des groupes suivants:

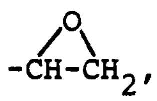 , 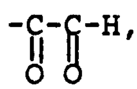 , 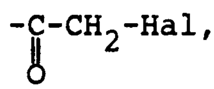 , ou 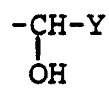

(a) (b) (c) (d)

où Hal représente le chlore, le brome ou l'iode et Y représente un groupe -COOH ou un derivé fonctionnel de celui-ci, avec un composé de formule (III) :

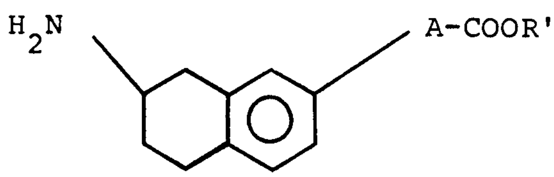

(III)

dans laquelle A est tel que défini ci-dessus et R' est un groupe ($C_1$-$C_4$)alkyle, et, quand -W est autre que (a), on traite le produit ainsi obtenu avec un agent réducteur approprié et quand on veut obtenir un composé de formule (I) dans laquelle R est un atome d'hydrogène, on hydrolyse le composé de formule (I) où R est R' ainsi obtenu.

Par agent réducteur approprié on entend un agent réducteur qui permet d'obtenir, au moins de préférence, la réduction séléctive de la chaîne entre le groupe amino et le cycle benzénique sans altération du groupe -COOR'.

Plus particulièrement, la réaction entre les composés de formule (II) et (III) est réalisée selon des modes opératoires et dans des conditions différentes qui sont essentiellement fonction de la nature du produit de départ de formule (II) utilisé et qui dépendent notamment de la signification du groupe -W.

Ces modes opératoires sont traités en détail ci-dessus et ont été designés avec la dénomination "Méthodes" allant de (a) à (d).

**Méthode (a)**

Selon ce mode opératoire, l'ouverture de l'époxyde de formule (IIa) :

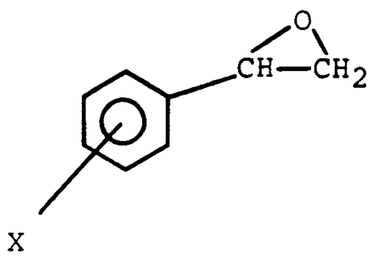

(IIa)

par l'amine (III) est conduite dans un solvant organique tel qu'un alcanol inférieur comme le méthanol, l'éthanol et l'isopropanol, le diméthylsulfoxyde, un éther linéaire ou cyclique, ou un amide comme le diméthylformamide ou le diméthylacétamide, en utilisant des quantités au moins équimoléculaires des réactifs, mais de préférence un excès de l'amine (III). La température de la réaction est comprise entre la température ambiante et la température de reflux du solvant choisi. Un agent de condensation basique, tel que l'hydroxyde de sodium ou l'acétate de sodium, peut être convenablement utilisé.

**Méthode (b)**

Dans la réaction qui comprend la condensation du phénylglyoxal de formule (IIb) :

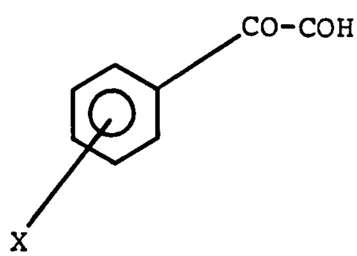

(IIb)

avec l'amine (III) et la réduction du produit obtenu, le mode opératoire préféré prévoit que les deux réactions soient conduites simultanémment en faisant réagir les composés (IIb) et (III) en présence d'un agent de réduction approprié, qui est en mesure de réduire le groupe oxo en un groupe hydroxy sans affecter le groupe -COOR'. Selon un mode opératoire approprié pour cette réaction, on utilise par exemple le borohydrure de sodium, dans un solvant alcoolique tel que l'éthanol, de préférence à basse température.

**Méthode (c)**

Selon un autre mode opératoire, les composés de formule (I) sont obtenus par réaction entre l'amine de formule (III) et une alpha-halocétophénone de formule (IIc) :

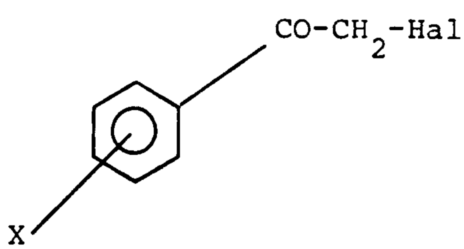

(IIc)

dans un solvant inerte, tel qu'un éther linéaire ou cyclique, un alcool inférieur comme le méthanol, l'éthanol ou l'isopropanol, un hydrocarbure aromatique comme le toluène ou le benzène, un hydrocarbure aliphatique halogéné comme le chloroforme, ou un nitrile comme l'acétonitrile. Cette réaction de substitution nucléophile est conduite avantageusement à la température ambiante ou à froid.

La réduction du produit ainsi obtenu peut être effectuée selon la méthode décrite au point (b).

**Méthode (d)**

Selon un autre mode opératoire, on fait réagir l'amine III avec un composé de formule (IId) :

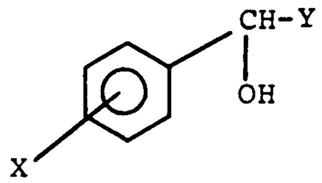

(IId)

dans laquelle X et Y sont tels que définis ci-dessus.

Comme dérivé fonctionnel du groupe -COOH, on peut utiliser le chlorure, l'anhydride, les anhydrides mixtes, les esters actifs ou l'acide libre convenablement activé, par exemple avec le dicyclohexylcarbodiimide (DCCI) ou l'hexafluorophosphate de benzotriazolyl-N-oxytris-(diméthylamino)-phosphonium (BOP). La réaction entre le composé de formule (IId) ci-dessus et l'aminotétraline (III) est conduite dans un solvant organique, aprotique, apolaire ou de préférence polaire, tel que le diméthylformamide, le diméthylsulfoxyde, le chlorure de méthylène, le benzène, le toluène, éventuellement en présence d'un accepteur de protons, tel que les amines tertiaires aliphatiques, notamment la triéthylamine.

Le mandélamide de formule suivante

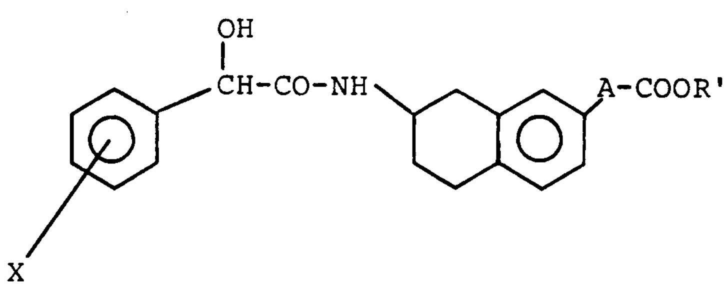

ainsi obtenu peut être directement soumis à une réduction du groupe amido en groupe méthylèneamino.

L'étape de réduction est effectuée, par exemple, par action du diborane, notamment d'un réactif générant le diborane tel que le complexe entre le borane et le di-méthylsulfure, ci-après désigné "borane-méthylsulfure", dans un solvant organique tel que le tétrahydrofuranne et en opérant à basse température (15-25 ° C), pour obtenir de préférence la réduction du groupe amido.

De préférence on utilisera la méthode (a) qui n'altère pas le groupe -COOR'.

Les composés de formule (I) dans laquelle R est un atome d'hydrogène peuvent ensuite être aisement préparés par saponification des esters correspondants.

Les produits (I) sont isolés selon les méthodes conventionnelles, de préférence sous forme d'un de leurs sels d'addition avec les acides minéraux ou organiques qui permettent une séparation ou une cristallisation convenable des composés comme indiqué ci-dessus, tels que l'acide picrique, l'acide oxalique ou un acide optiquement actif, par exemple un acide mandélique ou mandélique substitué, ou un acide camphorsulphonique, ou avec les acides minéraux ou organiques qui forment des sels pharmaceutiquement acceptables, tels que le chlorhydrate, le bromhydrate, le sulfate, l'hydrogénosulfate, le dihydrogénophosphate, le méthanesulfonate, le méthylsulfate, le maléate, le fumarate, le naphtalènesulfonate.

La base libre peut être libérée par neutralisation et transformée en un autre sel d'addition acide ou, lorsque R est un atome d'hydrogène, en un sel avec un métal, notamment alcalin ou alcalinoterreux, tel que le sel de sodium ou de calcium.

Les composés de formule (I) qui présentent les deux seuls atomes de carbone asymétrique marqués par l'astérisque peuvent exister sous quatre formes isomériques différentes. Le procédé de la présente invention permet d'opérer tant sur des mélanges racémiques que sur des isomères optiquement purs. Particulièrement, les réactions qui forment l'ensemble de ce procédé ne modifient pas la stéréochimie des composés concernés. Ainsi, en partant d'un composé de formule (IIb) ou (IIc), qui n'a pas de centre chiral, ou d'un composé de formule (IIa) ou (IId) sous forme racémique, et d'un composé de formule (III) sous forme racémique, on obtient un mélange d'isomères, notamment les isomères (R,R), (S,S), (R,S) et (S,R).

De même, en utilisant un composé de formule (III) sous forme optiquement active, par exemple ayant la configuration absolue R, on obtient un mélange de deux isomères seulement, à savoir le (R,R) et le (S,R).

Dans ce dernier cas, en utilisant aussi un composé de départ de formule (IIa) ou (IId) sous forme optiquement active, on peut obtenir les isomères purs.

Lorsqu'un mélange de quatre isomères est obtenu, il peut être séparé en deux couples d'énantiomères (R,R) + (S,S) et (R,S) + (S,R), diastéréoisomériques entre eux, par des techniques convenables comme la cristallisation fractionnée dans un solvant approprié, de préférence un alcanol inférieur, tel que l'éthanol, l'isopropanol ou leurs mélanges. Chaque couple d'énantiomères peut être ensuite séparé en les isomères purs par exemple par formation de sels diastéréoisomèriques, ou par chromatographie sur des colonnes chirales, ou par d'autres techniques convenables.

Lorsqu'un des composés de départ est sous forme optiquement active, le mélange des deux diastéréoisomères ainsi obtenu est séparé en les isomères purs selon les techniques citées précédemment.

Les composés de départ de formule (II) sont des produits connus, ou de toute façon ils peuvent être préparés selon des méthodes conventionnelles décrites dans la littérature chimique. Par exemple, les composés de formule (IIa) peuvent être obtenus par époxydation des dérivés styréniques correspondants par l'oxygène en présence d'un catalysateur à l'argent, ou par action du méthylide de diméthylsulfonium ou diméthylsulfoxonium sur le benzaldéhyde substitué correspondant selon la méthode décrite par E.J. Corey dans J. Am. Chem. Soc., 1956, 87, 1353.

Selon une méthode de préparation préférée, on peut obtenir un composé de formule (IIa) sous forme optiquement active par réduction de l'acide mandélique substitué ayant la configuration absolue voulue à l'atome de carbone en position alpha, en le dérivé glycolique correspondant, par estérification du groupe alcoolique primaire avec un derivé fonctionnel d'un acide sulfonique tel que le chlorure de tosyle ou de mésyle et, après, par cyclisation du composé ainsi obtenu par traitement avec une base forte telle qu'un hydroxyde alcalin dans les conditions de réaction conventionnellement utilisées pour les substitutions nucléophiles intramoléculaires.

Les composés de formule (IIb) sont aisément préparés par action d'un agent oxydant, tel que le dioxyde de sélénium, sur les acétophénones correspondantes, dans l'eau ou dans un solvant organique, tel qu'un éther cyclique, notamment le dioxanne ou le tétrahydrofuranne.

Selon un autre mode opératoire, lesdits composés de formule (IIb) sont préparés par action du diméthylsulfoxyde sur les halocétophénones de formule (IIc) ayant la même substitution sur le noyau benzénique selon la méthode décrite par N. Kornblum dans J. Am. Chem. Soc., 1957, 79, 6562, ou encore, à partir des alphadihaloacétophénones correspondantes par la réaction décrite par F. Venier dans le C.R. Acad. Sci., 1968, 266, 1650.

Les composés de départ de formule (IIc) sont aisément obtenus par halogénation des cétones correspondantes, ou dans certains cas, par une réaction de Friedel-Crafts en utilisant les dérivés benzéniques substitués correspondants et un halogénure d'un acide haloacétique. Enfin, les dérivés fonctionnels des acides mandélique ou mandéliques substitués de formule (IId) sont préparés à partir des acides correspondants qui, à leur tour, peuvent être obtenus par hydrolyse des mandélonitriles ayant la même substitution sur le noyau benzénique. Ces derniers composés peuvent être obtenus à partir soit du benzaldéhyde, substitué ou non, et de l'acide cyanhydrique, soit du benzaldéhyde, substitué ou non, du cyanure de sodium et du bisulfite de sodium selon des méthodes bien connues dans la littérature chimique. Les acides mandéliques de formule (IId) obtenus sous forme racémique peuvent être aisément séparés en leurs isomères optiquement purs par formation de sels diastéréoisomèriques avec des bases organiques optiquement actives appropriées selon les méthodes et les techniques conventionnelles.

Les composés de formule (III) et leurs sels éventuels, sont des produits nouveaux et représentent les intermédiaires clés dans la préparation des composés (I). Lesdits produits de formule (III) représentent, par conséquent, un autre objet de la présente invention. Des composés de formule (III) préférés comprennent les composés de formule (III) dans laquelle A représente un lien entre le groupe -COOR' et le noyau tétralinique, un groupe alkylène à 1 ou 2 atomes de carbone ou un groupe alcénylène à 2 atomes de carbone.

Les composés de formule (III) peuvent tous être préparés à partir d'un aldéhyde de formule (IV)

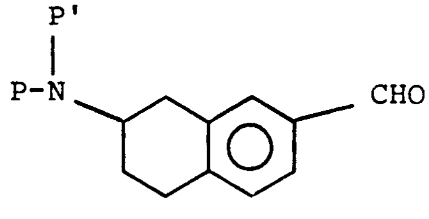

(IV)

dans laquelle P est un groupe protecteur temporaire du groupe amino convenablement choisi et P' est un atome d'hydrogène ou P et P' pris ensemble avec l'atome d'azote représentent un groupe protecteur du type phtalimido.

Pour obtenir les composés de formule (III) où A représente une liaison entre le groupe -COOR' et le noyau tétralinique, le composé de formule (IV) est oxydé, le groupe carboxylique libre est estérifié et le groupe protecteur est ensuite éliminé.

Pour la préparation des composés de formule (III) où A est un groupe méthylène, on fait réagir l'aldéhyde (IV) avec le chlorure de 2-(triméthylsilyl) éthoxyméthyl-triphénylphosphonium, on traite le produit intermédiaire ainsi obtenu de formule (V) :

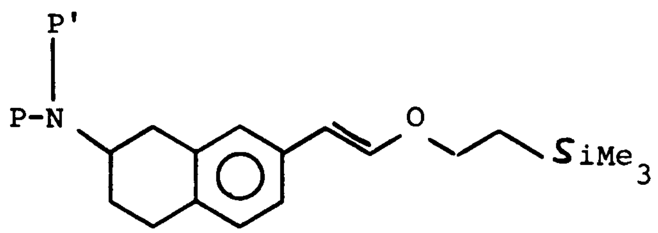

(V)

avec un fluorure de tétralkylammonium en suivant par la suite la procédure décrits ci-dessus mais à partir de l'aldéhyde de formule (VI) ainsi obtenu :

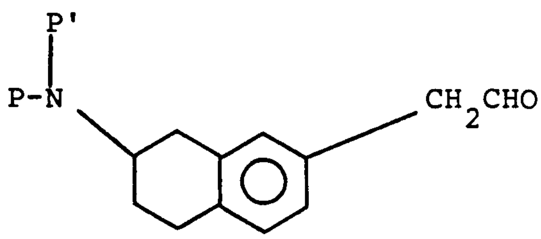

(VI)

Par réaction des aldéhydes (IV) ou (VI) avec les réactifs du type Wittig appropriés, qui contiennent déjà le substituant -COOR', suivie par une déprotection, on obtient directement les composés de formule (III) où A est un groupe ($C_2$-$C_4$)alcénylène et R' est tel que défini ci-dessus et, par réduction de la double liaison des produits ainsi obtenus, on arrive aux composés correspondants de formule (III) où A représente un groupe ($C_2$-$C_4$)alkylène.

On peut aussi convenablement obtenir des composés de formule (III) où A est un groupe ramifié, à partir des composés (III) obtenus selon les méthodes précédentes, comme indiqué ci-après : protection du groupe amino, mono- ou di-méthylation en position $\alpha$- par rapport au groupe -COOR', cette étape étant éventuellement suivie par l'hydrolyse du groupe ester, par la réaction de Arndt-Eistert, qui permet d'obtenir l'homologue supérieur le plus proche, et par l'estérification du groupe carboxylique, puis dans les deux cas déprotection du groupe amino ; on peut aussi obtenir lesdits composés a partir de l'aldéhyde (VI) par blocage du groupe -CHO (par exemple en en faisant une base de Schiff telle que la t-butylimine correspondante), mono- ou di-méthylation en position $\alpha$- par rapport au groupe -CHO, rétablissement de la fonction aldéhydique et réaction avec le réactif du type Wittig approprié, éventuellement suivie par la réduction de la double liaison.

Le choix d'un groupe protecteur approprié dépend donc du type de réaction qu'on prévoit à partir de l'aldéhyde (IV) et des conditions de réactions choisies.

Plus particulièrement, quand les réactions auxquelles l'aldéhyde (IV) est soumis ne prévoient pas l'utilisation de conditions fortement acides, les groupes protecteurs préféres seront les groupes t-alcoxycarbonyles tels que le t-butoxycarbonyle (BOC) ou le t-amyloxycarbonyle (AOC), le groupe BOC étant particulièrement préféré. L'élimination de ces groupes est aisement effectuée ensuite par hydrolyse acide selon des méthodes bien connues en littérature et notamment par action de l'acide trifluoroacétique ou de l'acide chlorhydrique dans un solvant alcoolique.

On peut aussi utiliser en tant que groupe protecteur un groupe benzyloxycarbonyle ou benzyloxycarbonyle substituée qui peut ensuite être éliminée par hydrogénation catalytique, de préférence en utilisant le palladium sur charbon comme catalyseur.

Enfin quand on prévoit l'utilisation de substances fortement nucléophiles on peut convenablement protéger le groupe amino en formant un derivé phtalimido qui est ensuite éliminé par traitement avec l'hydrazine ou une hydrazine substituée.

Il est toujours possible, si les conditions de réaction choisies l'exigent, d'utiliser comme produit de départ un aldéhyde (IV) protégé par un groupe protecteur donné, et d'éliminer ensuite ce groupe en le remplaçant par un groupe protecteur différent, lequel est ensuite éliminé comme indiqué plus haut.

L'homme du métier est, de toute facon , en mesure de choisir dans la littérature (voir par ex., D. Barton et W.C. Ollis, Comprehensive Organic Chemistry, Vol. 5, pp. 323-331 et les références que l'on y trouve citées) les groupes protecteurs appropriés dans les conditions de réaction prévues.

Plus particulièrement, l'oxydation de l'aldéhyde (IV), pour convertir le groupe -CHO en groupe carboxyle, peut être convenablement conduite à température ambiante en utilisant en tant qu'agent oxydant le réactif de Jones qui se compose d'une solution d'anhydride chromique dans de l'acide sulfurique dilué. La réaction est généralement conduite en ajoutant le réactif de Jones dans une solution de l'aldéhyde de départ dans l'acétone. L'acide ainsi obtenu est isolé selon des procédures tout-à-fait conventionnelles et estérifié par traitement avec un chloroformiate de $(C_1-C_4)$alkyle en présence d'une amine tertiaire qui sert d' accepteur de l'acide qui se forme dans la réaction. La réaction est de préférence conduite à une température comprise entre -10° et + 10°C, dans un solvant organique aprotique tel qu' un hydrocarbure halogéné. La déprotection du groupe amino est ensuite effectuée selon les méthodes classiques. Quand, selon un aspect préféré on utilise un groupe BOC, l'élimination de ce groupe est aisément effectuée par traitement d'une solution alcoolique du produit protégé avec une solution alcoolique d'acide chlorhydrique 4N.

La conversion de l'aldéhyde (IV) en aldéhyde (VI) est avantageusement effectuée en traitant l'aldéhyde (IV) avec une solution de chlorure de 2-(triméthylsilyl) éthoxyméthyl-triphénylphosphonium et d'un alcoolate alcalin dans un solvant organique aprotique notamment un éther alkylique ou cyclique tel que le tétrahydrofuranne ou le diméthoxyéthane, en séparant le produit intermédiaire de formule (V) par des méthodes conventionnelles et en le traitant avec un fluorure de tétraalkylammonium dans un solvant organique aprotique polaire qui peut convenablement contenir une très petite quantité d'eau.

Les aldéhydes de formules (IV) et (VI) peuvent donc être soumis à la réaction de Wittig modifiée par W.S. Wadsworth et W.D. Emmons (J. Amer. Chem. Soc. (1961), 83, 1733-38) en les faisant réagir avec un carboxyméthylphos phonate de formule $(EtO)_2P(O)CH(R'')-COOR'$, où R' est tel que defini ci-dessus et R'' est un atome d'hydrogène ou un groupe méthyle ou éthyle, en présence d'une base forte, telle que un hydrure alcalin, dans un solvant organique aprotique, genéralement le diméthoxyéthane ou le tétrahydrofuranne.

Quand le groupe -COOR' n'est pas lié directement à l'atome de carbone du phosphonate, on préfère utiliser le réactif de Wittig correspondant de formule $Ph_3P=CH-A'-COOR'$ où A' est un alkylène contenant 1 ou 2 atomes de carbone et R' est tel que défini ci-dessus, en présence généralement d'une base forte telle que le NaH ou le $(nC_4H_9)Li$, dans un solvant aprotique tel qu'un éther alkylique ou cyclique.

On obtient ainsi des composés de formule (III) où A est un groupe $(C_2-C_4)$alcénylène et R' est tel que défini ci-dessus. Si on le désire ces composés peuvent être transformés en les composés correspondants où A est un groupe $(C_2-C_4)$alkylène par réduction de la double liaison. Cette réduction peut être convenablement effectuée par hydrogénation catalytique à température et pression ambiantes en utilisant du palladium sur charbon en tant que catalyseur d'hydrogénation. Quand on utilise un groupe benzyloxycarbonyle ou benzyloxycarbonyle substitué en tant que groupe protecteur, on obtiendra donc dans la même étape l'élimination du groupe protecteur.

Si nécessaire, la mono- ou di-méthylation en position $\alpha$-par rapport au groupe carbalcoxy peut être achevée en traitant les composés (III) obtenus selon les méthodes indiquées plus haut , après protection du groupe amino, avec un halogénure de méthyle, généralemment $CH_3I$, en présence d'une base forte. On peut ensuite convertir les composés ainsi obtenus en leurs homologues supérieurs les plus proches par hydrolyse du groupe -COOR' en milieu basique, par la réaction de Arndt-Eistert (Ber., 1935, 68, 200) qui prévoit la conversion de l'acide en chlorure d'acide correspondant suivie par la réaction de ce dernier produit avec le diazométhane et par l'hydrolyse du produit ainsi obtenu en présence de $Ag_2O$, puis en effectuant l'estérification du groupe carboxyle, et enfin, la déprotection du groupe amino.

Quand on part de l'aldéhyde (VI), avant de le soumettre à la réaction de méthylation, il faudra bloquer le groupe aldéhydique en le transformant dans une base de Schiff, par exemple dans le dérivé tertbutylamino correspondant, par réaction avec une amine secondaire, par exemple la t-butylamine. On conduit donc l'alkylation de la même façon que ci-dessus, on rétablit l'aldéhyde par hydrolyse acide douce, on oxyde le groupe -CHO en groupe carboxylique, et on l'estérifie; ou on fait réagir cet aldéhyde avec le réactif de Wadsworth et Emmons et éventuellement on réduit la double liaison selon les techniques décrites ci-dessus.

Si on le désire, les mélanges racémiques des composés (III) ainsi obtenus sont séparés en isomères purs par formation des sels diastéréoisomériques avec des acides organiques optiquement actifs tels que les acides camphorsulfoniques, les acides mandéliques éventuellement substitués ou d'autres acides optiquement actifs.

On peut aussi hydrolyser l'ester et effectuer la séparation du mélange racémique en isomères optiquement purs par la formation de sels diastéréoisomériques avec des bases organiques optiquement actives, telles que les alpha-alkylbenzylamines, la menthylamine ou les autres bases utilisées dans les techniques conventionnelles.

Si l'aminotétraline (III) contient un deuxième centre chiral, les diastéréoisomères et les quatre isomères purs peuvent être isolés comme décrit ci-dessus. Ainsi, ils peuvent être utilisés pour la préparation de tous les isomères des composés (I).

L'aldéhyde (IV) à son tour est préparée à partir de la 2-amino-7-hydroxytétraline par un procédé en quatre étapes qui prévoit d'abord la protection du groupe amino, la réaction du produit avec l'anhydride trifluorométhanesulfonique, la conversion de la 2-amino-7- trifluorométhanesulfonyloxy-tétraline N-protégée ainsi obtenue en la 2-amino-7-vinyltétraline N-protégée correspondante par traitement avec le $[CH_3(CH_2)_3]_3SnCH=CH_2$ en présence de tétrakis(triphenylphosphine)palladium et l'oxydation du derivé vinylique avec un periodate alcalin catalysée par le tétraoxyde de osmium.

En partant d'un des isomères de la 2-amino-7-hydroxy-tétraline, produits connus dans la littérature et qui peuvent être préparés selon des méthodes déjà décrites, on arrive à l'isomère de l'aldéhyde (IV) ayant la même configuration absolue.

Les composés de formule (I) et leurs sels ont montrés des propriétés pharmacologiques très intéressantes en tant qu'agents antidépresseurs.

En particulier, les propriétés antidépressives des composés de formule (I) ont été étudiées dans le test de l'antagonisme aux effets hypothermisants de l'apomorphine, test habituellement utilisé en psycopharmacologie pour mettre en évidence une activité antidépressive chez l'homme.

On a aussi observé les modifications éventuelles du comportement et de l'activité motrice des souris traitées avec des doses croissantes des produits à étudier.

Les composés de formule (I) exercent des effets clairement indicatifs d'une activité antidépressive chez l'homme en antagonisant les effets hypothermisants exercés chez la souris par l'apomorphine à forte dose.

Les composés de formule (I) en outre ne produisent aucune sédation et aucune diminution de l'activité motrice spontanée chez les souris.

Le test a été conduit de la façon suivante :

- Antagonisme à l'hypothermie induite par l'apomorphine (Puech et al., Psychopharmacology, 75(1), 84-91, 1981).

On a utilisé des souris mâles CD1 (Charles River, France) d'un poids compris entre 22 et 25 g. Les souris sont placés en isolement dans des boites en matière plastique transparente. Les produits à étudier ont été mis en suspension dans une solution aqueuse à 1% de carboxyméthylcellulose. Les produits étaient administrés par voie intrapéritonéale sous le volume de 10 ml/kg (6 souris par dose).

Les animaux de contrôle reçevaient le véhicule seulement.

La température rectale a été mesurée à l'aide d'une sonde porteuse d'un couple thermoélectrique (relié à un galvanomètre) enfoncée à une profondeur constante.

Les produits à tester ainsi que le véhicule ont été administrés 30 minutes après la mesure de la température rectale de base et 30 minutes avant l'apomorphine (16 mg/kg s.c.). La température rectale a été mesurée à nouveau 30 minutes après l'administration d'apomorphine.

Les composés de formule (I) antagonisent les effets de l'apomorphine à partir de doses aussi faibles que celle de 0,03-1 mg/kg, i.p.

- Activité motrice

Aux mêmes doses, les composés de formule (I) n'ont provoqué presque aucune variation de l'activité motrice, les composés des Exemples 2 et 5 étant complétement inactifs même à la dose de 10 mg/kg i.p.

Les composés de formule (I) en outre, se sont montrés très actifs comme modulateurs de la motricité intestinale.

Leur aptitude à réduire la motricité spontanée du côlon a été observée dans des essais pharmacologiques normalisés "in vitro".

Dans les essais "in vitro", on a évalué la capacité des phényléthanolaminotétralines de l'invention à réduire, selon la concentration, la réponse contractile, dans des conditions normalisées particulières, des bandes de côlon proximal de rats isolés.

On sacrifie des rats mâles, non à jeun, pesant de 250 à 300 g. La partie proximale du côlon, consistant en un segment de 2 à 3 cm environ, est prélevée et mise en suspension dans une cuve à organes de 20 ml contenant une solution de Krebs-Ringer ayant la composition mM suivante : NaCl 118,4; KCl 4,7; $CaCl_2$ 2,45; $MgSO_4$ 1,16; $NaH_2PO_4$ 3,7; Glucose 5,6; $NaHCO_3$ 30,9. La solution est aérée par un mélange d'oxygène (95 %) et de $CO_2$ (5%) et sa température maintenue constante à 37° C. Les segments du côlon, soumis à une traction de 1 g environ, se contractent spontanément. Les composés à l'étude sont ajoutés après stabilisation de la préparation (2h).

On détermine la $CE_{50}$ qui représente la concentration du composé qui produit une réduction de 50% de la contraction du tissu atteinte en l'absence de composés à tester.

Dans ce test, les composés de la présente invention ont montré une activité très élevée, caractérisée, pour les produits les plus actifs, par des $CE_{50}$ de l'ordre de 0,5 à 50 nM.

Les composés de formule I ont montré aussi une spécificité surprenante pour le côlon. Des essais "in vitro", effectués selon la même méthode générale, mais sur l'uterus isolé de rat, ont montré qu'on obtient un effet significatif sur la contractilité spontanée de l'uterus à des doses beaucoup plus élevées que celles actives sur le côlon.

Les composés de formule (I) et leurs sels pharmaceutiquement acceptables possèdent en outre une toxicité très faible, compatible avec l'utilisation de ces produits en tant que médicaments.

Ainsi, la présente invention, selon un autre de ses aspects, concerne des compositions pharmaceutiques renfermant, en tant que principe actif, un ou plusieurs composés de formule (I) ci-dessus ainsi que leurs sels pharmaceutiquement acceptables.

Les compositions pharmaceutiques de la présente invention peuvent être préparés pour l'administration orale, sublinguale, sous-cutanée, intramusculaire, intraveineuse, transdermique, ou rectale des principes actifs de formule (I), de préférence sous formes unitaires d'administraton, en mélange avec des supports pharmaceutiques classiques.

Ces formes unitaires de dosage contiennent généralement de 0,1 à 500 mg et de préférence de 0,5 à 250 mg de principe actif par unité de dosage.

Des formes unitaires de dosage appropriées pour l'administration orale comprennent les comprimés, les gélules, les poudres, les granules et les solutions ou suspensions orales.

Lorsqu'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'elixir ou pour l'administration sous forme de gouttes peut contenir le principe actif avec un édulcorant , acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcolorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Le principe actif principal de formule (I) peut être administré sous forme de base libre ou de sel pharmaceutiquement acceptable tel quel ou sous forme de complexe avec, par exemple, une cyclodextrine ou bien en association ou en co-administration avec d'autres principes actifs.

Les exemples suivants illustrent l'invention sans toutefois la limiter. Les solvants indiqués entre parenthèses après le point de fusion sont les solvants de cristallisation.

Le terme "tétraline" se référe au noyau 1,2,3,4-tétrahydronaphtalène.

## PREPARATION I

**Chlorhydrate de 2-amino-7-(2-éthoxycarbonyl)vinyltétraline.**

**a)** 2-tertbutoxycarbonylamino-7-trifluorométhanesulfonyloxy-tétraline.

On refroidit à 0 ° C un mélange de 24 g (0,09 mole ) de 2-tertbutoxycarbonylamino-7-hydroxytétraline et 50 ml de pyridine sous atmosphère d'azote et on y ajoute 16,4 ml (0,10 mole ) d'anhydride trifluorométhanesulfonique. Après 10 minutes à 0 ° C, on porte la température du mélange réactionnel à la valeur ambiante et on la maintient à cette température pendant 2,5 heures. On verse dans l'eau et on extrait à l'éther éthylique.

On lave la phase organique avec une solution d'acide chlorhydrique 1N et après à l'eau, on sèche sur sulfate de sodium et on concentre sous vide.

Le produit huileux foncé ainsi obtenu est purifié par chromatographie sur colonne de silice en éluant avec un mélange éther de pétrole : acétate d'éthyle 9:1.

On obtient ainsi 25 g du produit indiqué ci-dessus. P.f. 87-89 ° C

**b)** 2-tertbutoxycarbonylamino-7-vinyltétraline.

On chauffe à la température de reflux pendant 3 heures un mélange de 25 g (0,063 mole ) du produit obtenu à l'étape a), 280 ml de dioxanne anhydre, 19,3 ml (0,066 mole ) de tributyl-vinylétain, 8,141 g de LiCl, 1,483 g de $Pd(Ph_3P)_4$) et quelques cristaux de 2,6-ditertbutyl-4-méthyl-phénol, sous atmosphère d'azote. On refrodit à la température ambiante et on y ajoute 32 ml de pyridine et 90 ml d'une solution 1M de florure de tetrabutylammonium dans du tétrahydrofuranne.

On laisse à température ambiante pendant 16 heures, on y ajoute de l'éther éthylique et on filtre sur célite.

On lave la solution organique à l'eau, avec une solution 1N d'acide chlorhydrique et encore à l'eau.

On sèche sur sulfate de sodium et on concentre sous vide. Le produit huileux ainsi obtenu est purifié par chromatographie sur colonne de silice en éluant avec cyclohexane : acétate d'éthyle 9:1. On obtient ainsi 10 g de 2-tertbutoxycarbonylamino-7-vinyltétraline. P.f. 104-105 ° C. (éther isopropylique)

**c)** 7-formyl-2-tertbutoxycarbonylaminotétraline.

On agite pendant 3 heures à température ambiante et sous atmosphère d'azote, un mélange de 2,5 g (0,009 mole ) du produit obtenu à l'étape b), 6 g de periodate de sodium, 3,3 ml d'une solution à 2,5% de tetroxyde d'osmium dans le tertbutanol, 60 ml de tétrahydrofuranne et 20 ml d'eau. On le verse dans de l'eau et on extrait avec de l'acétate d'éthyle. La solution organique est lavée à l'eau, séchée sur sulfate de sodium et concentrée sous vide. On purifie le produit huileux foncé ainsi obtenu par chromatographie sur colonne de silice en éluant avec un mélange éther de pétrole : acétate d'éthyle 95:5. On obtient une huile qui solidifie en quelques instants et qui est cristallisée de l'éther isopropylique. Rendement : 1,7 g.

**d)** 2-tertbutoxycarbonylamino-7-(2-éthoxycarbonyl)vinyltétraline.

On agite pendant 1 heure à température ambiante et sous atmosphère d'azote un mélange de 0,6 g (0,024 mole ) d' hydrure de sodium à 80%, 4,8 ml de diéthylphosphonacétate d'éthyle et 50 ml de diméthoxyéthane et après on y ajoute goutte à goutte une solution de 3,4 g du produit obtenu à l'étape c) dans 65 ml de diméthoxyéthane. Après 4 heures à température ambiante, on ajoute de l'eau et on extrait par de l'éther éthylique. On lave la phase organique à l'eau, on la séche sur sulfate de sodium et on la concentre sous vide. On obtient 3,1 g d'une huile jaune pâle qui est purifiée par chromatographie sur colonne de silice en éluant d'abord avec un mélange cyclohexane : acétate d'éthyle 9:1 et après avec un mélange 8:2 des mêmes solvants. Rendement: 2,7 g. P.f. 110-111 ° C (éther isopropylique).

**e)** Chlorhydrate de 2-amino-7-(2-éthoxycarbonyl)vinyltétraline.

On laisse réagir à température ambiante pendant 5 heures une solution de 2,7 g (0,0078 mole ) du produit obtenu à l'étape d) dans 50 ml d'éthanol absolu et 33 ml d'une solution 4N d'acide chlorhydrique dans l'éthanol absolu.

On concentre sous vide et on séche à l'étuve en obtenant 2,05 g du produit indiqué dans le titre.

P.f. 184-186 ° C

**PREPARATION II**

**Chlorhydrate de 2-amino-7-(2-éthoxycarbonyl)éthyltétraline**

**a)** 2-tertbutoxycarbonyl-7-(2-éthoxycarbonyl)éthyl-tétraline

On charge dans un appareil d'hydrogénation 2,6 g (0,075 mole ) du composé obtenu dans la Préparation I, étape d), 60 ml d'éthanol absolu et 0,26 g de palladium sur charbon à 5%. On hydrogène à la température ambiante et à la pression ordinaire et après 2,5 heures on filtre sur célite et on concentre le filtrat sous vide en obtenant 2,2 g de 2-tertbutoxycarbonyl-7-(2-éthoxycarbonyl)éthyltétraline.
P.f. 110-11 ° C.

**b)** Chlorhydrate de 2-amino-7-(2-éthoxycarbonyl)éthyltétraline.

On fait réagir à la température ambiante pendant 5 heures un mélange de 2,1 g (0,006 mole ) du composé obtenu à l'étape a) ci-dessus, 40 ml d'éthanol absolu et 25 ml d'une solution 4N d'acide chlorhydrique en éthanol absolu. On concentre sous vide et on séche à l'étuve le solide blanc ainsi obtenu. Rendement : 1,7 g. P.f. 162-164 ° C.

**PREPARATION III**

**Chlorhydrate de 2-amino-7-éthoxycarbonyltétraline**

**a)** 7-éthoxycarbonyl-2-tertbutoxycarbonylamino-tétraline.

On charge dans un ballon de réaction 1,55 g du produit obtenu à l'étape c) de la Préparation I et 15 ml d'acétone, et on y ajoute, en maintenant la température à 20 ° C, 8 ml du réactif de Jones (une solution d'anhydride chromique dans de l'acide sulfurique dilué). Après 5,5 heures on y ajoute de l'isopropanol et on filtre. On concentre sous vide, on reprend par de l'eau et on y ajoute NaOH pour rendre la solution aqueuse basique. On extrait par de l'éther éthylique, on acidifie la phase aqueuse avec HCl et on l'extrait deux fois par de l'éther éthylique. Les extraits organiques sont combinés, lavés à l'eau, séchés sur sulfate de sodium et concentrés sous vide en obtenant 1,5 g d'un produit solide jaune pâle.

On refroidit à 0 ° C un mélange du produit ainsi obtenu avec 20 ml de chlorure de méthylène et 0,79 ml de triethylamine, et on y ajoute 0,55 ml (0,0057 mole ) de chloroformiate d'éthyle, et, 5 minutes plus tard, 0,32 g de diméthylaminopyridine. On maintient à 0 ° C pendant 30 minutes, on verse dans de l'eau et on y ajoute du chlorure de méthylène.

La phase organique est séparée, lavée successivement avec une solution aqueuse de bicarbonate de sodium, une solution aqueuse 1N d'acide chlorhydrique et à l'eau. On la sèche sur sulfate de sodium et on concentre sous vide en obtenant une huile qui est purifiée par chromatographie sur colonne de silice en éluant d'abord avec un mélange cyclohexane : acétate d'éthyle 9:1, et après avec un mélange des mêmes solvants en rapport 8:2. Rendement : 0,52 g.
$^1$H RMN (80 MHz,$CDCl_3$) (m,2H), 7(m, 1H), 4,25(q, 2H), 4,4(1H,-NH-), 3,8(m, 1H), 1,3(m, 12H).

**b)** Chlorhydrate de 2-amino-7-éthoxycarbonyl-tétraline.

On fait réagir, à température ambiante, pendant 24 heures, un mélange de 0,5 g (0,0016 mole ) du composé obtenu à l'étape a) ci-dessus, 10 ml d'éthanol absolu et 7 ml d'une solution 4N d'acide chlorhydrique dans l'éthanol. On concentre sous vide en obtenant une huile jaune pâle qui solidifie dans l'isopropanol. On filtre et on obtient 0,29 g du composé indiqué dans le titre.
$^1$H RMN (80 MHz, DMSO-$d_6$) $\delta$:8,4(-$NH_2$ HCl), 7,6(m, 2H), 7,1(m, 1H), 4,2(q, 2H), 1,3(t, 3H).

**PREPARATION IV**

**Chlorhydrate de (2R)2-amino-7-(2-éthoxycarbonyl)vinyltétraline**

**a)** (2R) 7-hydroxy-2-tertbutoxycarbonylamino-tétraline.

On refroidit à 0°C une suspension de 9,4 g (0,058 mole ) de (2R)2-amino-7-hydroxytétraline dans 39,5 ml (0,280 mole ) de triéthylamine et 100 ml de N,N-diméthylformamide anhydre. On y ajoute 13,6 g (0,063 mole ) de ditertbutyldicarbonate et on laisse le mélange réactionnel à température ambiante pendant 2 heures. On verse dans 500 ml d'eau, on extrait avec de l'éther éthylique, on lave à l'eau, on sèche et on évapore à sec. On purifie le produit ainsi obtenu par chromatographie flash en éluant avec un mélange cyclohexane : acétate d'éthyle 7:3, en obtenant 12,7 g du composé indiqué ci-dessus $[\alpha]_D^{20}$ = + 69,3° (c = 1%, MeOH).

**b)** (2R)2-tértbutoxycarbonylamino-7-trifluorométhane-sulfonyloxy-tétraline.

On refroidit à 0°C un mélange de 12,4 g (0,047 mole ) du composé obtenu à l'étape a) ci-dessus, et 25 ml de pyridine sous atmosphère d'azote et on y ajoute, pendant 25 minutes, 8,5 ml (0,052 mole ) d'anhydride trifluorométhane-sulfonique, en maintenant la température entre 0 et 5°C. On agite pendant 2 heures et demie à température ambiante et on verse dans 400 ml d'eau. On extrait avec de l'éther éthylique, on sèche, on filtre et on évapore à sec. On purifie le produit ainsi obtenu par chromatographie flash en éluant avec un mélange cyclohexane : acétate d'éthyle 85: 15, et en refroidissant le produit huileux que l'on obtient par évaporation des fractions le contenant.
Rendement : 15 g. P.f. 52-55°C
$[\alpha]_D^{20}$ = + 48.3° (c = 1%, MeOH).

**c)** (2R) 2-tert-butoxycarbonylamino-7-vinyltétraline.

On suit la procédure décrite dans la Préparation I mais en partant du produit obtenu ci-dessus. Rendement 7 g. P.f. 89-91°C (éther isopropylique). $[\alpha]_D^{20}$ = + 40° (c = 0,3%, $CH_2Cl_2$).

**d)** (2R) 7-formyl-2-tert-butoxycarbonylaminotétraline.

On suit la procédure décrite dans la Préparation I, étape c), mais en partant du produit obtenu dans l'étape c) ci-dessus et en purifiant le produit ainsi obtenu par chromatographie flash avec un mélange hexane/acétate d'éthyle 70/30. Rendement 4,9 g. P.f. 98-100°C (éther isopropylique), $[\alpha]_D^{20}$ = + 41,5°(c = 0,3%, $CH_2Cl_2$)

**e)** (2R) 7-(2-éthoxycarbonyl)vinyl-2-tertbutoxycarbonylaminotétraline.

En suivant la procédure décrite dans la Préparation I, étape d), mais en partant du produit obtenu ci-dessus, on obtient 3,3 g du composé indiqué ci-dessus. P.f. 107-108°C (cyclohexane). $[\alpha]_D^{20}$ = + 35,2°(c = 0,3%, $CH_2Cl_2$).

**f)** Chlorhydrate de (2R) 2-amino-7-(2-éthoxycarbonyl)vinyltétraline.

En suivant la procédure décrite dans l'étape e) de la Préparation I, mais à partir du produit obtenu à l'étape e) ci-dessus on obtient le produit indiqué dans le titre.
P.f. 204-206°C; $[\alpha]_D^{20}$ = + 52.3°(c = 1%, MeOH)

## PREPARATION V

**Chlorhydrate de (2R) 7-(2-éthoxycarbonyl)éthyl-2-aminotétraline.**

**a)** (2R) 7-(2-éthoxycarbonyl)éthyl-2-tertbutoxycarbonylaminotétraline.

En suivant la procédure de la Préparation II, étape a) en partant du composé obtenu dans la Préparation IV, étape e), (0,75 g, 0,0021 mole ), on obtient 0,7 g du composé indiqué ci-dessus.
P.f. 101-102°C.
$[\alpha]_D^{20}$ = + 30,3° (c = 0,3%, $CH_2Cl_2$).

**b)** Chlorhydrate de (2R) 7-(2-éthoxycarbonyl)éthyl-2-aminotétraline.

En suivant essentiellement la procédure de la Préparation II, étape b) mais en partant de 0,6 g (0,0017

mole ) du composé obtenu dans l'étape a) ci-dessus, on obtient 0,4 g du composé indiqué dans le titre.
P.f. 175-178° C (acétone).
$[\alpha]_D^{20}$ = + 45,6° C (c = 0,3%, $CH_2Cl_2$)

**PREPARATION VI**

**(2S) 2-amino-7-(2-éthoxycarbonyl)vinyltétraline**

On obtient le produit indiqué dans le titre (p.f. 200-203° C (acétone)), en suivant la procédure de la Préparation IV mais à partir de la (2S) 2-amino-7-hydroxytétraline, et en passant par les intermédiaires suivants :

**a)** (2S) 7-hydroxy-2-tertbutoxycarbonylaminotétraline
$[\alpha]_D^{20}$ = -62,4° (c = 1%, MeOH)
**b)** (2S) 2-tertbutoxycarbonylamino-7-trifluorométhane-sulfonyloxytétraline
$[\alpha]_D^{20}$ = -62,4° (c = 1%, MeOH)
**c)** (2S) 2-terbutoxycarbonylamino-7-vinyltétraline
P.f. 90-92° C (éther isopropylique)
$[\alpha]_D^{20}$ = -38,1° (c = 0,3% $CH_2Cl_2$)
**d)** (2S) 7-formyl-2-tertbutoxycarbonylaminotétraline
P.f. 98-101° C (éther isopropylique)
$[\alpha]_D^{20}$ = -45,3° (c = 0,3%, $CH_2Cl_2$)
**e)** (2S) 7-(2-éthoxycarbonyl)vinyl-2-tertbutoxycarbonylaminotétraline
P.f.106-108° C (éther isopropylique)
$[\alpha]_D^{20}$ = -36,1° (c = 0,3%, $CH_2Cl_2$)

**PREPARATION VII**

**Chlorhydrate de (2S) 2-amino-7-(2-éthoxycarbonyl)éthyl-tétraline.**

**a)** (2S) 7-(2-éthoxycarbonyl)éthyl-2-tertbutoxycarbonyl-aminotétraline.

En suivant la procédure de la Préparation II étape a) mais en partant du composé obtenu dans la Préparation VI, on obtient le composé indiqué ci-dessus. P.f. 100-102° C (éther isopropylique); $[\alpha]_D^{20}$ = -30,7° (c = 0,3%, $CH_2Cl_2$)

**b)** chlorhydrate de (2S) 2-amino-7-(2-éthoxycarbonyl)éthyl-tétraline.

On suit la procédure de la Préparation II, étape b), mais en partant du composé obtenu dans l'étape a) ci-dessus, et on obtient le produit indiqué dans le titre.
Rendement 60%. P.f. 178-180° C
$[\alpha]_D^{20}$ = -44,8° (c = 0,3%, $CH_2Cl_2$)

**PREPARATION VIII**

**Chlorhydrate de 2-amino-7-(3-éthoxycarbonyl)allyltétraline**

**a)** 7-formylméthyl-2-tertbutoxycarbonylamino-tétraline.

On refroidit à -60° C sous atmosphère d'azote un mélange de 1,1 g (9,9 mmoles) de tert-butylate de potassium dans 130 ml de tétrahydrofuranne anhydre et on y ajoute 4,7 g (10,8 mmoles) de chlorure de 2-(triméthylsilyl) éthoxyméthyltriphenylphosphonium. Après 10 minutes a -60° C, on laisse monter la température et on agite pendant 10 minutes. On y ajoute goutte à goutte une solution de 1 g (3,6 mmoles) du composé obtenu dans la Préparation Ic) dans 10 ml de tétrahydrofuranne. On agite pendant 20 minutes et on verse dans 200 ml d'eau et glace. On extrait à l'éther éthylique, on sèche sur sulfate de sodium, on filtre et on concentre sous vide. On purifie le résidu ainsi obtenu par chromatographie flash, en éluant avec un mélange de cyclohexane et acétate d'éthyle 9:1. On obtient 1,2 g d'un produit huileux jaune qui correspond à la 2-tertbutoxycarbonylamino-7-[2-(2-(triméthylsilyl)éthoxy)vinyl]-tétraline.

A une solution de 1 g du produit ainsi obtenu (2,56 mmoles) dans 19,5 ml d'acétonitrile et 0,5 ml d'eau,

on ajoute 20 ml d'une solution 1,1 M du fluorure de tétrabutylammonium.

On chauffe à 80 ° C pendant 30 minutes sous atmosphère d'azote, on réfroidit et on verse dans 150 ml d'eau. On extrait à l'éther éthylique, on sèche les extraits sur sulfate de sodium, on filtre et on concentre à sec. Le résidu est purifié par chromatographie flash en éluant avec un mélange cyclohexane:acétate d'éthyle 8:2. On obtient 100 mg du produit indiqué ci-dessus.

**b)** 7-(3-éthoxycarbonyl)allyl-2-tertbutoxycarbonylamino-tétraline.

En suivant la procédure de la Préparation I d) mais à partir du composé obtenu à l'étape a) ci-dessus, on obtient le produit indiqué.

**c)** Chlorhydrate de 2-amino-7-(3-éthoxycarbonyl)allyl-tétraline.

On obtient le produit indiqué dans le titre en suivant la procédure décrite dans la Préparation I e) mais à partir du composé obtenu ci-dessus à l'étape b).

## PREPARATION IX

**Chlorhydrate de 2-amino-7-(3-éthoxycarbonyl)propyltétraline**

**a)** 7-(3-éthoxycarbonyl)propyl-2-tertbutoxycarbonylamino-tétraline.

En opérant comme décrit dans la Préparation II a) mais à partir du produit obtenu dans la Préparation VIII b) on obtient le produit indiqué ci-dessus.

**b)** Chlorhydrate de 2-amino-7-(3-éthoxycarbonyl)propyl-tétraline.

On obtient le produit indiqué dans le titre en suivant la procédure décrite dans la Préparation II b) mais à partir du composé obtenu dans l'étape a) ci-dessus.

## PREPARATION X

**Chlorhydrate de (2R) 2-amino-7-éthoxycarbonyltétraline**

**a)** (2R) 7-éthoxycarbonyl-2-tertbutoxycarbonylamino-tétraline.

On ajoute une solution de 920 mg de $NaClO_2$ à 80% (8,13 mmoles) et 730 mg de $NaHPO_4 . 5 H_2O$ (5,3 mmoles) dans 5 ml d'eau à une solution de 200 mg de (2R) 7-formyl-2-tert-butoxy carbonylaminotétraline obtenue à la préparation IV, étape d) (0,73 mmoles) dans 7 ml de tert-butanol.

On agite énergiquement à température ambiante pendant 1 heure, on ajoute une solution 1N d'acide chlorhydrique jusqu'à pH environ 3 et on extrait à l'acétate d'éthyle. On sèche la phase organique sur du sulfate de sodium, on filtre et on évapore à sec. On dissout le résidu ainsi obtenu dans un mélange de 10 ml de chlorure de méthylène et 0,11 ml de triéthylamine, on réfroidit la solution à 0 ° C et on y ajoute goutte à goutte 80 µl d'éthyl chloroformiate et, environ 5 minutes plus tard, 50 ng de 4-diméthylamino-pyridine. On laisse le mélange réactionnel à 0 ° C pendant 1 heure et après on le verse dans l'eau et on extrait à l'acétate d'éthyle. On lave la phase organique successivement avec une solution 1N d'acide chlorhydrique, à l'eau, avec une solution saturée de bicarbonate de sodium, et à l'eau. On sèche sur sulfate de sodium, on filtre et on évapore à sec. On purifie le résidu ainsi obtenu par chromatographie flash en éluant avec un mélange cyclohexane/éthyl acétate 75/25. On obtient ainsi 200 mg du produit indiqué ci-dessus.
P.f. 125-128 ° C.

**b)** Chlorhydrate de (2R) 2-amino-7-éthoxycarbonyltétraline

On ajoute 7 ml d'une solution 8,4 N d'acide chlorhydrique dans de l'éthanol à une solution de 600 mg du produit obtenu à l'étape a) ci-dessus (1,9 mmoles) dans 20 ml d'éthanol et on laisse le mélange réactionnel à température ambiante pendant 5 heures. On évapore à sec, on reprend le résidu en le triturant dans 10 ml d'acétone chaud, on refroidit et on filtre en obtenant 350 mg du produit indiqué dans le titre. P.f. 185-188 ° C.

$[\alpha]_D^{20}$ = + 54,8° (c = 0,3%, MeOH).

**PREPARATION XI**

**Chlorhydrate de (2S) 2-amino-7-éthoxycarbonyltétraline.**

On obtient le produit indiqué dans le titre (p.f. 189-191 °C;$[\alpha]_D^{20}$ = - 52,4°(c = 0,3%, MeOH)) en suivant la procédure de la préparation X mais à partir de la (2S) 7-formyl-2-tert- butoxycarbonylaminotétraline obtenue dans la Préparation VI, étape d), et en passant par l'intermédiaire suivant :

**a)** (2S) 7-éthoxycarbonyl-2-tertbutoxycarbonylamino-tétraline,p.f. 124-125° C

**EXAMPLE 1**

**Chlorhydrate de N-(7-éthoxycarbonyl)-1,2,3,4-tétra-hydronapht-2-yl)-2-hydroxy-2-(3-chlorophenyl)-éthanamine.**

On chauffe à 80° C pendant 24 heures, sous atmosphère d'azote, un mélange de 0,2 g (92 mmoles) de chlorhydrate de 2-amino-7-(2-éthoxycarbonyl)éthyltétraline, 2 ml de diméthylsulfoxyde et 0,21 g (1,34 mmoles) de l'oxyde du 3-chlorostyrène. On verse dans de l'eau et on extrait à l'acétate d'éthyle. On lave la phase organique à l'eau, on séche sur sulfate de sodium et on concentre sous vide en obtenant un produit huileux jaune foncé qui est converti en le chlorhydrate correspondant par traitement avec de l'acide chlorhydrique gaseux dans l'isopropanol. Le produit ainsi obtenu est isolé par filtration et cristallisé dans un mélange isopropanol/éthanol 9/1.

On obtient ainsi 0,12 g du composé indiqué dans le titre.
P.f. 199-201° C (Isopropanol/éthanol 9/1).

**EXEMPLE 2**

**Chlorhydrate de N-[7-(2-éthoxycarbonyl)éthyl-1,2,3,4-tétrahydronapht-2-yl]-2-(3-chlorophenyl)-éthanamine.**

On chauffe à 80° C pendant 16 heures un mélange de 1,2 g (0,005 mole ) du produit obtenu dans la Préparation II mais sous forme de base libre, 6 ml de diméthylsulfoxyde et 1,17 g (0,0073 mole ) de l'oxyde du 3-chlorostyrène oxyde. On verse le mélange réactionnel dans l'eau et on extrait à l'acétate d'éthyle. On lave la phase organique à l'eau et on la sèche sur sulfate de sodium. On concentre sous vide en obtenant un produit huileux jaune pâle qui est purifié par chromatographie sur colonne de silice en éluant avec de l'acétate d'éthyle. On dissout le produit ainsi obtenu dans de l'éther éthylique et on y ajoute de l'éthanol saturé d'acide chlorhydrique gaseux. On isole le produit qui précipite par filtration en obtenant 1,07 g du composé indiqué dans le titre. P.f. 145-148° C (éthanol).

**EXAMPLES 3-4**

En suivant la procédure de l'Exemple 2 mais à partir du composé obtenu dans la Préparation IV et des oxydes chiraux du 3-chlorostyrène on obtient les composés suivants :

**Exemple 3**

Chlorhydrate de N-[(2R) 7-(2-éthoxycarbonyl)éthyl-1,2,3,4-tétrahydronapht-2-yl]-(2R)-2-hydroxy-2-(3-chlorophenyl)-éthanamine.
P.f. 138-140° ($Et_2O$)
$[\alpha]_D^{20}$ = + 31,5° (c = 1%, MeOH)

**Exemple 4**

Chlorhydrate de N-[(2R) 7-(2-éthoxycarbonyl)éthyl-1,2,3,4-tétrahydronapht-2-yl]-(2S)-2-hydroxy-2-(3-chlorophenyl)-éthanamine.

**EXAMPLES 5-6**

En suivant la procédure de l'Exemple 2 mais à partir du composé obtenu dans la Préparation VII et des 3-chlorostyrène oxydes chiraux on obtient les composés suivants.

**Exemple 5**

Chlorhydrate de N-[(2S) 7-(2-éthoxycarbonyl)éthyl-1,2,3,4-tétrahydronapht-2-yl]-(2R)-2-hydroxy-2-(3-chlorophenyl)-éthanamine.
P.f. 164-166 ° C (éther isopropylique)
$[\alpha]_D^{20}$ = -78,5 ° (c = 1%, MeOH)

**Exemple 6**

Chlorhydrate de N-[(2S) 7-(2-éthoxycarbonyl)éthyl-1,2,3,4-tétrahydronapht-2-yl]-(2S)-2-hydroxy-2-(3-chlorophenyl)-éthanamine.

**EXAMPLE 7**

**Chlorhydrate de N-[7-(2-carboxy)éthyl-1,2,3,4-tétrahydronapht-2-yl]-2-hydroxy-2-(3-chlorophenyl)-éthanamine.**

On dissout 450 mg du composé de l'Exemple 1 (1,1 mmole) dans 3 ml d'éthanol absolu et on y ajoute 2 ml de NaOH 1N. On agite à température ambiante pendant 3-4 heures, on évapore l'éthanol, on dissout le résidu dans de l'eau et on lave la solution aqueuse à l'éther éthylique. On ajoute de l'acide chlorhydrique 1N à la phase aqueuse jusqu'à pH acide, on filtre le précipité et on le sèche en obtenant 100 mg du composé indiqué dans le titre.
P.f. 152-155 ° C.
I.R. (KBr) : 1703 $cm^{-1}$ (-COOH) et 3350 $cm^{-1}$ (-OH)-Spectrométrie de masse : E.I. $M^+$ = 411; F.A.B. $MH^+$ = 374

**EXAMPLE 8**

**Chlorhydrate de N-[7-(2-carbéthoxy)vinyl-1,2,3,4-tétrahydronapht-2(S)-yl]-(2R)-2-hydroxy-2-(3-chlorophenyl)-éthanamine.**

On chauffe à reflux pendant 30 heures un mélange de 200 mg du composé obtenu dans la Préparation VI mais sous forme de base libre (0,71 mmoles) et 152 mg du (R)-3-chlorostyrene oxyde (0,98 mmoles) dans 5 ml d'éthanol. On évapore le solvant et on purifie le résidu ainsi obtenu par chromatographie flash sur une colonne de gel de silice en éluant avec un mélange chlorure de méthylène/méthanol 9/1. On prépare le chlorhydrate du produit ainsi obtenu dans de l'isopropanol en obtenant 150 mg du composé indiqué dans le titre.
P.f. 117-120 ° C.
$[\alpha]_D^{20}$ = -104 ° (c = 0,3%, MeOH)

**EXAMPLE 9**

**Chlorhydrate de N-[7-(3-carbéthoxy)propyl-1,2,3,4-tétrahydronapht-2-yl]-2-hydroxy-2-(3-chlorophényl)éthanamine.**

En suivant la procédure décrite dans l'Exemple 1 mais à partir du composé de la Préparation IX b) on obtient le produit indiqué dans le titre.

**EXAMPLE 10**

**Chlorhydrate de N-[7-(2-carbéthoxy)vinyl-1,2,3,4-tétrahydronapht-2(R)-yl]-(2R)-2-hydroxy-2-(3-chlorophényl)-éthanamine.**

En suivant la méthode décrite dans l'Exemple 8 mais à partir du produit obtenu dans la Préparation IV f) sous forme de base libre et de l'oxyde du (R)-3-chlorostyrene, on obtient le produit indiqué dans le titre.
P.f. 205-207 ° C.
$[\alpha]_D^{20}$ = + 56,4 ° (0,5%, MeOH)

**EXAMPLE 11**

**Chlorhydrate de N-[7-(2-carbéthoxy)vinyl-1,2,3,4-tétrahydronapht-2-yl]-2-hydroxy-2-(3-chlorophényl)-éthanamine.**

En suivant la méthode de l'Exemple 8 mais à partir du produit obtenu dans la Préparation I e) sous forme de base libre et de l'oxyde de 3-chlorostyrène, on obtient le produit indiqué dans le titre.
P.f. 149-151 ° C.

## Revendications

**1.** Une phényléthanolaminotétraline de formule (I)

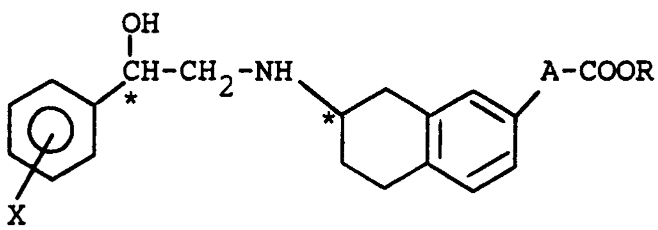

(I)

dans laquelle
X représente un atome d'hydrogène, un atome d'halogène, un groupe ($C_1$-$C_4$)alkyle, ou un groupe trifluorométhyle,
A représente une liaison entre le groupe -COOR et le noyau tétralinique, un groupe ($C_1$-$C_4$)-alkylène, ou un groupe ($C_2$-$C_4$)alcénylène, et
R représente un atome d'hydrogène ou un groupe ($C_1$-$C_4$)alkyle,
ou un de ses sels.

**2.** Une phényléthanolaminotétraline selon la revendication 1 où X représente un atome d'hydrogène ou un atome d'halogène ou un groupe trifluorométhyle en position 3 ou un de ses sels.

**3.** Une phényléthanolaminotétraline selon la revendication 1 ou 2 où A représente une liaison entre le groupe -COOR et le noyau tétralinique, un groupe alkylène à 1 ou 2 atomes de carbone, ou un groupe alcénylène à 2 atomes de carbone, ou un de ses sels.

**4.** Une phényléthanolaminotétraline selon la revendication 1 où X est un atome d'halogène en position 3-, A représente une liaison entre le groupe -COOR et le noyau tétralinique, ou un groupe alkylène à 1 ou 2 atomes de carbone, et R représente un atome d'hydrogène ou un groupe méthyle ou éthyle, ou un de ses sels.

**5.** Un procédé de préparation d'un composé de formule (I)

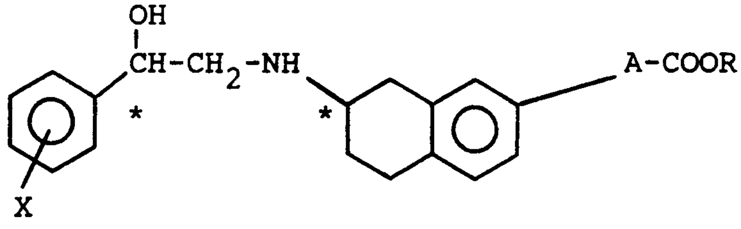

(I)

dans laquelle

X représente un atome d'hydrogène, un atome d'halogène, un groupe $(C_1-C_4)$alkyle ou un groupe trifluorométhyle,

A représente une liaison entre le groupe -COOR et le noyau tétralinique, un groupe $(C_1-C_4)$-alkylène, ou un groupe $(C_2-C_4)$alcénylène, et

R représente un atome d'hydrogène ou un groupe $(C_1-C_4)$alkyle,

ou d'un des ses sels, caractérisé en ce qu'on fait réagir un composé de formule (II)

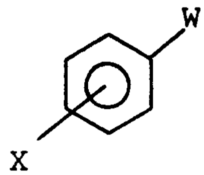

(II)

dans laquelle X est tel que defini ci-dessus et le radical -W représente un des groupes suivants :

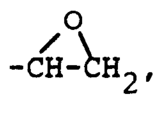 , 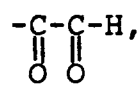 , 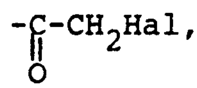 , ou 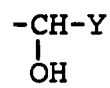

(a) (b) (c) (d)

où Hal représente le chlore, le brome ou le iode et Y représente un groupe -COOH ou un derivé fonctionnel de celui-ci, avec un composé de formule (III)

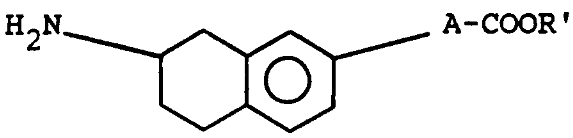

(III)

dans laquelle A est tel que defini ci-dessus et R' est un groupe $(C_1-C_4)$alkyle et, quand -W est autre que (a), on traite le produit ainsi obtenu avec un agent réducteur approprié et si on veut obtenir un composé de formule (I) dans laquelle R est un atome d'hydrogène, on hydrolyse le composé de formule (I) où R est R', ainsi obtenu, et éventuellement on transforme le composé de formule (I) dans un de ses sels selon des méthodes par elles-mêmes connues.

**6.** Procédé selon la revendication 5 caractérisé en ce que le radical -W représente un groupe (a).

**7.** Composition pharmaceutique caractérisée en ce qu'elle comprend en tant que principe actif au moins un composé de formule (I) dans laquelle X, A et R sont tels que definis dans la revendication 1 ou un de ses sels pharmaceutiquement acceptables.

**8.** Un composé de formule (III)

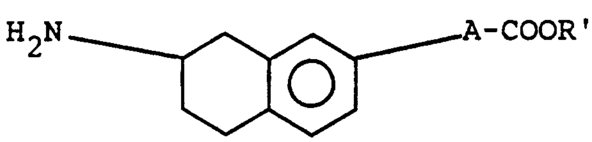

(III)

dans laquelle

A représente une liaison entre le groupe -COOR' et le noyau tétralinique, un groupe $(C_1-C_4)$-alkylène, ou un groupe $(C_2-C_4)$alcénylène et

R' représente un groupe $(C_1-C_4)$alkyle, ainsi que ses sels et ses dérivés N-protégés.

**9.** Un composé selon la revendication 8 où A représente une liaison entre le groupe -COOR' et le noyau tétralinique, un groupe alkylène à 1 ou 2 atomes de carbone ou un groupe alcénylène à 2 atomes de carbone.

**10.** Les isomères purs de l'aldéhyde de formule (IV)

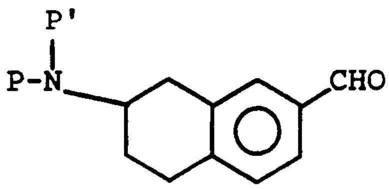

(IV)

dans laquelle P est un groupe protecteur temporaire du groupe amino et P' est un atome d'hydrogène ou P et P' pris ensemble avec l'atome d'azote représentent un groupe protecteur du type phtalimido.

Office européen des brevets

# RAPPORT DE RECHERCHE EUROPEENNE

Numero de la demande

EP 91 40 0415

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | EP-A-0 211 721 (MIDY S.p.A.)<br>* Revendications *<br>--- | 1-7 | C 07 C 229/50<br>A 61 K 31/24<br>A 61 K 31/95<br>C 07 C 271/24 |
| A,D | EP-A-0 383 686 (MIDY S.p.A.)<br>* Revendications *<br>--- | 1-7 | |
| A | WO-A-9 101 297 (BOEHRINGER INGELHEIM GmbH)<br>* Page 12; exemple 12; revendications *<br>----- | 1-9 | |
| | | | **DOMAINES TECHNIQUES RECHERCHES (Int. Cl.5)** |
| | | | C 07 C 229/00<br>A 61 K 31/00 |

Le présent rapport a été établi pour toutes les revendications

| Lieu de la recherche | Date d'achèvement de la recherche | Examinateur |
|---|---|---|
| LA HAYE | 16-10-1991 | SANCHEZ Y GARCIA J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant